# EUROPEAN PATENT SPECIFICATION

(11) **EP 4 609 797 B1**
(45) Date of publication and mention of the grant of the patent: **14.10.2026**
(21) Application number: 25160016.9
(22) Date of filing: 25.02.2025
(51) Int. Cl.: A61B 6/46, A61B 6/58, G06F 3/01

(54) **INFORMATION PROCESSING APPARATUS, INFORMATION PROCESSING METHOD, AND INFORMATION PROCESSING PROGRAM**
INFORMATIONSVERARBEITUNGSVORRICHTUNG, INFORMATIONSVERARBEITUNGSVERFAHREN UND INFORMATIONSVERARBEITUNGSPROGRAMM
APPAREIL, PROCÉDÉ ET PROGRAMME DE TRAITEMENT D'INFORMATIONS

(30) Priority: 01.03.2024 JP 2024031605
(43) Date of publication of application: 03.09.2025
(73) Proprietor: FUJIFILM Corporation, Tokyo 106-8620 (JP)
(72) Inventor: SASADA, Ryoji, Tokyo, 106-8620 (JP)
(74) Representative: Dehns Germany Partnerschaft mbB

(56) References cited:
- WO-A1-2017/006535
- JP-A- 2014 064 960
- US-A1- 2015 253 865
- US-A1- 2018 206 810

## Description

### BACKGROUND OF THE INVENTION

### 1. Field of the Invention

The present invention relates to an information processing apparatus, an information processing method, and an information processing program.

### 2. Description of the Related Art

JP2014-064960A discloses an X-ray imaging apparatus comprising an X-ray tube that irradiates a subject with X-rays, a collimator that adjusts an X-ray emission field, a visible light source for visually recognizing the emission field with visible light, and a body thickness measuring device that measures a body thickness of the subject, in which the X-ray imaging apparatus further comprises a light source control device that turns off or turns on the visible light source in a case where a change in an output signal of the body thickness measuring device exceeds a determined threshold value.

JP2021-010541A discloses an X-ray diagnostic apparatus comprising an X-ray tube device that emits X-rays toward a subject, and an X-ray diaphragm device that comprises an X-ray diaphragm blade limiting an irradiation region of the X-rays applied to the subject, in which the X-ray diaphragm device comprises an irradiation field illumination unit that emits irradiation light for illuminating an irradiation range of the X-rays, an X-ray shielding box that accommodates the X-ray diaphragm blade and the irradiation field illumination unit and that has an opening serving as a passage for the X-rays and the irradiation light, and a light emitting unit that is disposed on an outer peripheral surface of the X-ray shielding box on an opening side.

WO2017/006535A discloses a radiography apparatus comprising a radiation irradiation device that irradiates a subject with radiation, imaging means that images the subject to acquire a captured image of the subject, a radiation detector that detects the radiation transmitted through the subject to generate a radiation image of the subject, and a driving situation controller that controls a driving situation of at least one of the radiation irradiation device or the radiation detector according to whether or not the radiation detector is present in the captured image. In addition, WO2017/006535A discloses the radiography apparatus that determines a part of the subject included in the captured image, and does not turn on an irradiation field lamp via a halfway push-operation of an imaging button in a case where the part is an animal face.

WO2020/115967A discloses an X-ray imaging apparatus comprising an X-ray irradiator that irradiates an imaging object with X-rays, light that irradiates the imaging object with visible light to indicate a range of the X-rays emitted from the X-ray irradiator, and a switch mechanism that is installed in a housing constituting the X-ray irradiator and that switches turning on and turning off the light, in which the switch mechanism comprises an insulating plate that is fixed to the housing in a state of penetrating an inside and an outside of the housing and that has a surface serving as the outside of the housing and a rear surface serving as the inside of the housing, an antenna that is installed on the rear surface of the insulating plate, and a controller that is connected between the antenna and the light and that controls at least one of turning on or turning off the light based on a change amount in capacitance of the antenna.

JP2011-245274A discloses an X-ray system comprising an imaging system including an X-ray radiation source, an X-ray image receptor, and a control circuit for controlling the X-ray radiation source, and a handheld interface device configured to wirelessly communicate with the imaging system, in which the handheld interface device is configured to receive a user input command for operating the imaging system, and the imaging system is configured to wirelessly receive the command and execute the command to operate the imaging system.

### SUMMARY OF THE INVENTION

In radiography such as X-raying, a user such as a radiologist who operates a radiography apparatus turns on light provided in the radiography apparatus to determine a range in which a subject is irradiated with radiation. In a case where the irradiation range is determined, the user can acquire a radiation image of the subject by turning off the light and irradiating the subject with the radiation.

An object of the present disclosure is to provide an information processing apparatus that can easily control turning on and turning off light remotely, as compared with a case where the light in a case of determining a range in which radiation is emitted is turned on and turned off by a switch.

In order to achieve the above object, an information processing apparatus according to a first aspect comprises a processor, in which the processor is configured to: acquire a real-time video with an imaging apparatus; and control at least one of turning on or turning off light used to determine an irradiation range of radiation according to a movement of an object included in the acquired video.

In an information processing apparatus according to a first embodiment, in the information processing apparatus according to the first aspect, in a case where a presence of a person is detected as the movement of the object from the acquired video, the processor controls the light to be turned on.

In an information processing apparatus according to a second embodiment, in the information processing apparatus according to the first aspect, in a case where a movement of a person is detected as the movement of the object from the acquired video, the processor controls the light to be turned on.

In an information processing apparatus according to a third embodiment, in the information processing apparatus according to the first aspect, in a case where a specific movement of a person is detected as the movement of the object from the acquired video, the processor controls the light to be turned on or turned off.

In an information processing apparatus according to a fourth embodiment, in the information processing apparatus according to the first aspect, in a case where a movement of a plurality of persons is detected as the movement of the object from the acquired video, the processor recognizes a specific movement of a specific person from among the plurality of persons, and controls the light to be turned on or turned off based on the recognized specific movement of the specific person.

In an information processing apparatus according to a fifth embodiment, in the information processing apparatus according to the first aspect, the processor is configured to: limit a range in which the video is acquired to a size of an image receiving unit for the radiation or to the irradiation range of the radiation; and control turning on and turning off the light based on the video acquired in the limited range.

In an information processing apparatus according to a sixth embodiment, in the information processing apparatus according to any one of the first aspect or first to fifth embodiments, the processor is configured to control turning on and turning off the light based on a first mode in which the light is turned on and turned off using the acquired video or on a second mode in which the light is not turned on and turned off using the acquired video.

In an information processing apparatus according to a seventh embodiment, in the information processing apparatus according to the sixth embodiment, the processor is configured to control turning on and turning off the light via the first mode in a state in which radiography is possible.

In an information processing apparatus according to an eighth embodiment, in the information processing apparatus according to the sixth embodiment, the processor is configured to control turning on and turning off the light via the first mode in a case of radiography in a specific imaging menu.

In an information processing apparatus according to a ninth embodiment, in the information processing apparatus according to the sixth embodiment, the processor is configured to control turning on and turning off the light via the first mode in a case of radiography at an upright position.

In an information processing apparatus according to a tenth embodiment, in the information processing apparatus according to the sixth embodiment, the video acquired by the imaging apparatus is a visible light image.

In an information processing apparatus according to an eleventh embodiment, in the information processing apparatus according to the sixth embodiment, the video acquired by the imaging apparatus is a visible light image and a depth image corresponding to the visible light image.

An information processing program according to a second aspect causes a computer to execute a process comprising: acquiring a real-time video with an imaging apparatus; and controlling at least one of turning on or turning off light used to determine an irradiation range of radiation according to a movement of an object included in the acquired video.

An information processing method according to a third aspect is executed by a computer, the method comprising: a step of acquiring a real-time video with an imaging apparatus; and a step of controlling at least one of turning on or turning off light used to determine an irradiation range of radiation according to a movement of an object included in the acquired video.

With the information processing apparatus of the first aspect, an effect is exhibited that a user can easily control turning on and turning off light remotely, as compared with a case where the user turns on and turns off the light in a case of determining a range in which radiation is emitted by a switch.

### BRIEF DESCRIPTION OF THE DRAWINGS

Fig. 1 is a diagram showing an outline of a radiography system according to the present embodiment.
Fig. 2 is a diagram showing a schematic configuration of an imaging apparatus according to the present embodiment.
Fig. 3 is a diagram showing a hardware configuration of a controller and a console according to the present embodiment.
Fig. 4 is a flowchart showing an example of a process executed by an information processing program.
Fig. 5 is a flowchart showing an example of a control process of a light irradiation unit via a first mode.
Fig. 6 is a flowchart showing an example of the control process of the light irradiation unit via a second mode.

### DESCRIPTION OF THE PREFERRED EMBODIMENTS

Hereinafter, embodiments of the present disclosure will be described in detail with reference to the accompanying drawings.

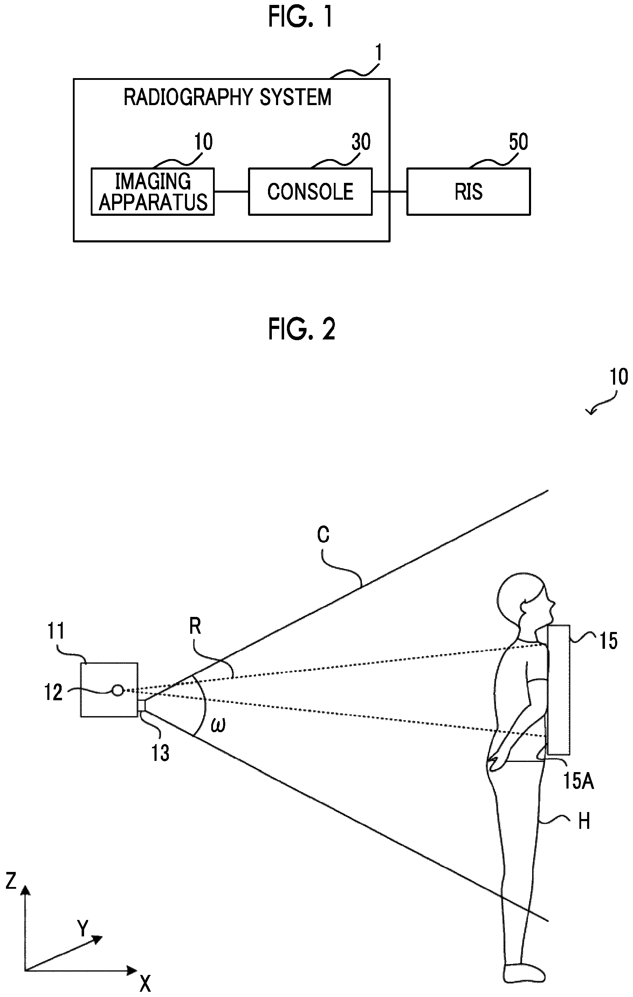

Fig. 1 is a diagram showing a schematic configuration of a radiography system 1. As shown in Fig. 1, the radiography system 1 comprises an imaging apparatus 10 and a console 30. The imaging apparatus 10 and the console 30, and the console 30 and an external radiology information system (RIS) 50 are communicatively connected to each other via a wired or wireless network.

The console 30 acquires the imaging order or the like from the RIS 50. In addition, the console 30 controls the imaging apparatus 10 according to the acquired imaging order, the setting and the instruction designated by the user, and the like, and performs the radiography process. In a case where the radiography process is started, the imaging apparatus 10 performs the radiography in which a subject H is irradiated with radiation under the control of the console 30 and a radiation image of the subject H is acquired. The console 30 is an example of an "information processing apparatus".

The console 30 is operated by a person who handles the imaging apparatus 10, for example, a radiologist. The radiologist is an example of a "user". The radiography system 1 may include picture archiving and communication systems (PACS) (not shown).

Next, the imaging apparatus 10 will be explained with reference to Fig. 2. Fig. 2 is a diagram showing a schematic configuration of the imaging apparatus 10. Fig. 2 shows an example of a state in which the chest of the patient indicating the subject H is radiographed as the imaging part.

As shown in Fig. 2, the imaging apparatus 10 comprises a radiation emitting unit 11 and a radiation detector 15. The radiation emitting unit 11 includes a radiation source 12 that emits radiation such as X-rays, and an optical camera 13. The optical camera 13 is an example of an "imaging apparatus". In addition, the radiation detector 15 is an example of an "image receiving unit".

In a case where the user gives an instruction to emit the radiation, the imaging apparatus 10 emits the radiation from the radiation source 12. A range R shown in Fig. 2 indicates a region where radiation is emitted. The range R is adjusted to be changeable by the user. The type of the radiation source 12 is not particularly limited, and for example, a radiation source such as a hot cathode type or a cold cathode type can be appropriately applied.

The optical camera 13 is an optical digital camera that includes, for example, a complementary metal oxide semiconductor (CMOS) image sensor or a charge coupled device (CCD) image sensor and that performs imaging based on visible light. As the optical camera 13, a 2D camera such as a Color camera, a 3D camera comprising a Color camera and a Depth camera, or the like can be appropriately applied. The optical camera 13 enables the still image capture and the motion picture capture. The optical camera 13 can image the periphery of the radiation emitting unit 11 and acquire a visible light image, a video of a depth image corresponding to the visible light image, and the like in real time.

In the radiography system 1, the movement of a person, an object, and the like included in the image and the video acquired by the optical camera 13 can be detected using, for example, an inter-frame difference, optical flow, a posture estimation system, and the like.

In addition, in the radiography system 1, the presence of the person, the object, and the like included in the image and the video acquired by the optical camera 13 can be detected using, for example, a human body detection system, a posture estimation system, and the like.

The optical camera 13 can image a region wider than the range R shown in Fig. 2, for example, a range C shown in Fig. 2.

The position at which the optical camera 13 is installed is not particularly limited. For example, the optical camera 13 shown in Fig. 2 may be attached to the same surface as the irradiation opening for radiation of the radiation emitting unit 11. Alternatively, the optical camera 13 may be attached to a wall surface or the like of the imaging room.

The radiation detector 15 detects the radiation transmitted through the subject H on a detection surface 15A. The radiation detector 15 generates the radiation image based on the detected radiation, and outputs the generated radiation image to, for example, the radiation emitting unit 11 and the console 30.

The radiation detector 15 is, for example, a portable electronic cassette, and may be used by being placed on any seat or being held by the subject H. In addition, the radiation detector 15 may be movable to any position in the horizontal direction (the X direction and the Y direction) and the vertical direction (the Z direction) with respect to the radiation emitting unit 11. In addition, the radiation detector 15 may be a stationary type to be disposed inside an imaging table installed in the imaging room.

The type of the radiation detector 15 is not particularly limited. The radiation detector 15 may be an indirect-conversion-type radiation detector that converts the radiation into light and converts the converted light into a charge, or may be a direct-conversion-type radiation detector that directly converts the radiation into a charge.

The radiation emitting unit 11 may be a ceiling-running type emitting unit that is held by a support column suspended from a ceiling of an imaging room. In the ceiling-running type emitting unit, a support column that is expandable and contractible in the vertical direction (Z direction) is attached to rails running around the ceiling via wheels, and the emitting unit is movable in the horizontal direction (X direction and Y direction) in the imaging room. By the movement of the support column in the horizontal direction and the expansion and contraction of the support column in the vertical direction, the radiation emitting unit 11 is also translationally moved in the horizontal direction and the vertical direction. In addition, the radiation emitting unit 11 may be movable rotationally around a rotational axis extending in the horizontal direction, or may be movable rotationally around a rotational axis extending in the vertical direction.

In addition, the radiation emitting unit 11 may be, for example, a portable emitting unit. The portable emitting unit may be used for, for example, a simple radiographic examination in a medical facility, a radiographic examination in a case of home medical care, a radiographic examination outdoors, an on-site medical care in a disaster-stricken area or a medically underserved area, or the like. In addition, for example, the radiation emitting unit 11 may be a stationary emitting unit installed in an imaging room. The operation of the radiation emitting unit 11 is controlled by the console 30.

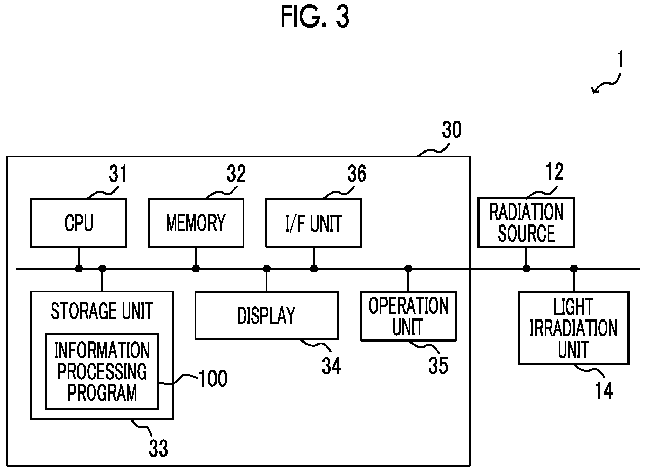

Fig. 3 is a diagram showing an example of a hardware configuration of the console 30 that controls the operation of the radiation emitting unit 11 and the operation of the imaging apparatus 10. For description, Fig. 3 also shows a part of a hardware configuration of the imaging apparatus 10.

The console 30 includes a central processing unit (CPU) 31, a memory 32 such as a read only memory (ROM) or a random access memory (RAM), a storage unit 33, a display 34, an operation unit 35, an interface (I/F) unit 36, and the like.

The CPU 31, for example, expands various programs including an information processing program 100 stored in the storage unit 33 to the memory 32 and controls each configuration of the radiation emitting unit 11.

The display 34 displays the image and the video captured by the optical camera 13 in real time. The display 34 may display the radiation image generated in the radiation detector 15.

The I/F unit 36 is an interface for connecting each configuration provided in the imaging apparatus 10 and the console 30 to each other in a communicable manner. In addition, the I/F unit 36 is an interface for connecting the console 30 and the RIS 50 in a communicable manner.

The CPU 31 can control turning on and turning off a light irradiation unit 14 provided in the radiation emitting unit 11. The user can turn on the light irradiation unit 14 to determine the position of the range R that is the irradiation region of the radiation, and the like.

The light irradiation unit 14 is provided with, for example, a timer (not shown). After the light irradiation unit 14 is started to be turned on, the timer starts counting, and after a predetermined time such as 30 seconds elapses, the light irradiation unit 14 is automatically turned off. The light irradiation unit 14 is an example of "light". The light irradiation unit 14 is composed of light emitting diode (LED) light, laser diode (LD) light, and the like.

The operation unit 35 is an interface between each component provided in the console 30 and the imaging apparatus 10 and the user. The turning on and turning off of the light irradiation unit 14 can be controlled by, for example, a switch provided in the console 30, the imaging apparatus 10, and the like.

In the console 30, the information on the patient can be acquired from the RIS 50, and the acquired information on the patient can be displayed on the display 34. The user can input information on a patient or the like serving as the subject H in the console 30 and can acquire the information on the patient from the RIS 50. In addition, the CPU 31 is an example of a "processor".

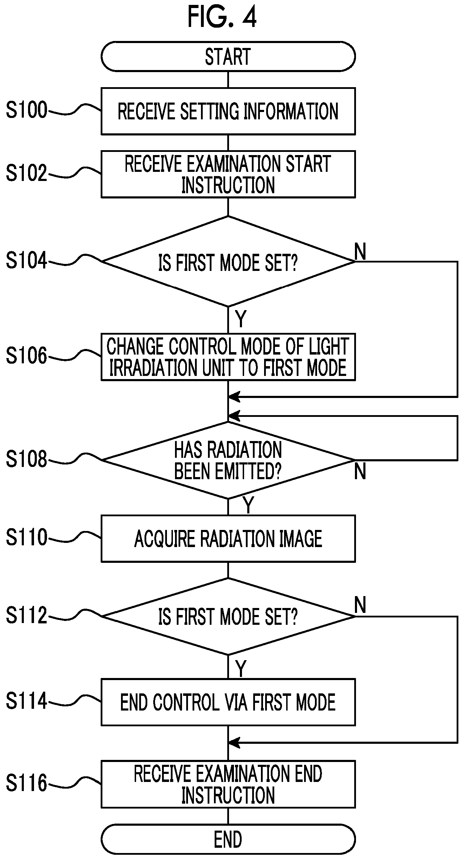

Next, a process of the information processing program 100 executed by the CPU 31 will be described with reference to Fig. 4.

Fig. 4 is a flowchart showing an example of a process executed by the CPU 31 with the information processing program 100.

In step S100, the CPU 31 receives the setting information from the user. Here, the setting information includes various settings in the imaging apparatus 10 in a case of imaging the subject H, such as an imaging menu and a light control mode for controlling turning on and turning off the light irradiation unit 14. The setting information is registered by the user. In addition, the setting information includes information on a patient serving as the subject H. The setting information is registered in, for example, the console 30.

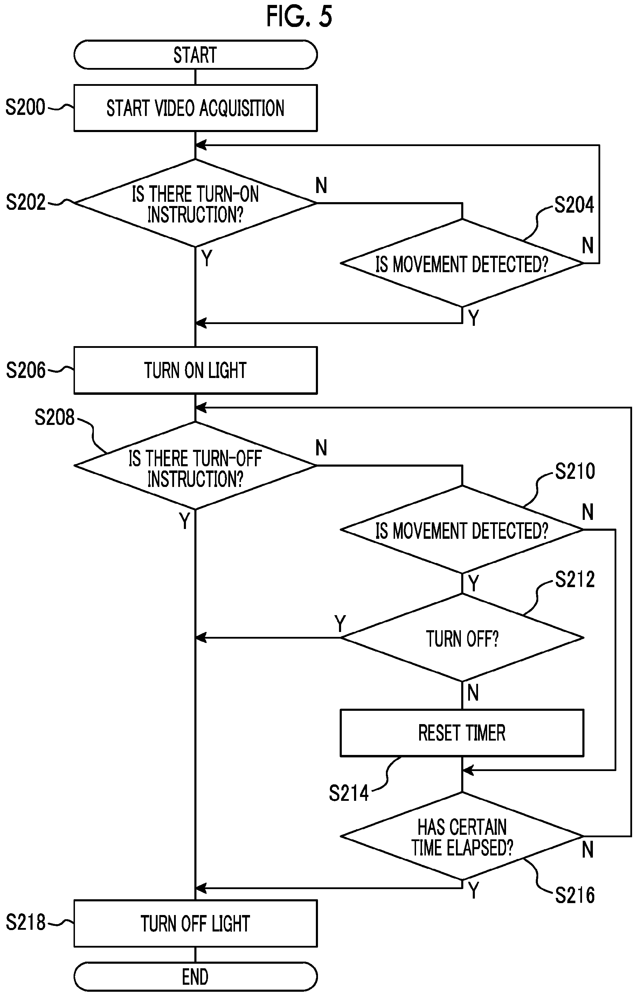

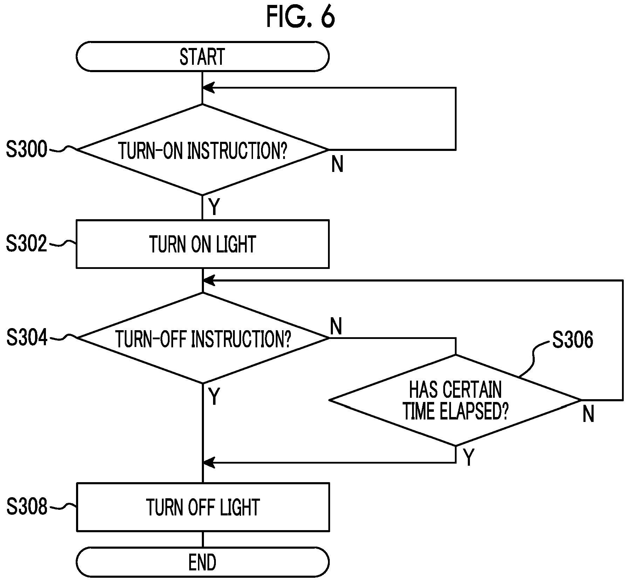

In the imaging menu, a part of the patient serving as the subject H to be imaged, such as the head, the chest, the abdomen, and the leg, can be registered. In the light control mode, it is possible to select and register whether or not to use the first mode as the light control mode for controlling turning on and turning off the light irradiation unit 14. According to the light control in the first mode, it is possible to control turning on and turning off the light using the video acquired from the optical camera 13. Details of the control of the light irradiation unit 14 in the first mode will be described later (see Fig. 5). The user can control turning on and turning off the light irradiation unit 14 by operating the operation unit 35 regardless of whether or not the first mode is set. A mode in which the turning on and turning off of the light irradiation unit 14 are controlled by a mode other than the first mode, in other words, the turning on and turning off of the light irradiation unit 14 are controlled by the operation of the operation unit 35 without using the video, will be hereinafter referred to as a second mode. Details of the control of turning on and turning off the light irradiation unit 14 via the second mode will be described later (see Fig. 6).

In step S102, the CPU 31 receives an instruction to start the examination. The instruction to start the examination is given, for example, by the user operating the console 30. In a case where the instruction to start the examination is received, the CPU 31 starts the radiography process with the radiography system 1.

In step S104, the CPU 31 determines whether or not the first mode is set as the control mode of the light irradiation unit 14 in the setting information. In a case where the first mode is set, the CPU 31 affirms the determination. In a case where the determination is affirmed, the CPU 31 executes the process of step S106. In a case where the first mode is not set, the CPU 31 denies the determination, and executes the process of step S108.

In step S106, the CPU 31 changes the control mode of the light irradiation unit 14 to the first mode.

In step S108, the CPU 31 determines whether or not the radiation has been emitted. The irradiation instruction of the radiation is given by the user. In a case where the radiation is emitted, the CPU 31 affirms the determination of step S108. In a case where the emission of the radiation has not been completed, the CPU 31 denies the determination of step S108.

In a case where the determination of step S108 is affirmed, the CPU 31 executes the process of step S110. In a case where the determination of step S108 is denied, the CPU 31 executes the process of step S108.

In step S110, the CPU 31 acquires the radiation image generated in the radiation detector 15.

In step S112, the CPU 31 determines whether or not the control mode of the light irradiation unit 14 is set to the first mode. The case where the control mode of the light irradiation unit 14 is set to the first mode is, for example, a case where the determination is affirmed in the process of step S104. In a case where the control mode of the light irradiation unit 14 is set to the first mode, the CPU 31 affirms the determination of step S112 and executes the process of step S114. In a case where the control mode of the light irradiation unit 14 is not set to the first mode, the CPU 31 denies the determination of the process of step S112 and executes the process of step S116.

In step S114, the CPU 31 ends the control of the light irradiation unit 14 via the first mode.

In step S116, the CPU 31 receives an instruction to end the examination, for example, via an instruction from the user. After the instruction to end the examination is received, the CPU 31 ends the radiography process with the radiography system 1. The CPU 31 executes the process shown in Fig. 4 each time the radiography is performed.

With the above process, in a case where the control mode of the light irradiation unit 14 is set to the first mode, the CPU 31 can control the light irradiation unit 14 in the first mode, for example, while executing the process from step S106 to step S110. Under the control of the light irradiation unit 14 via the first mode, the user can determine a range in which the radiation is emitted and can acquire the radiation image of the subject H.

Next, a detailed description of process of controlling the light irradiation unit 14 via the first mode will be made with reference to Fig. 5. The process shown in Fig. 5 is executed by the CPU 31, for example, in a case where the determination of step S104 of Fig. 4 is affirmed.

In step S200, the CPU 31 acquires the video captured by the optical camera 13 in real time.

In step S202, the CPU 31 determines whether or not a turn-on instruction indicating an instruction to turn on the light irradiation unit 14 has been received by the operation of the operation unit 35 of the user. In a case where the turn-on instruction is received by the operation of the operation unit 35 of the user, the CPU 31 affirms the determination and executes the process of step S206. In a case where the operation unit 35 is not operated and the turn-on instruction is not received by the operation unit 35, the CPU 31 denies the determination and executes the process of step S204.

In the process of step S204, the CPU 31 determines whether or not at least one of the presence or movement of the object or the person included in the acquired video is detected.

Specifically, for example, in a case where the presence of the object or the person is detected in the acquired video, the CPU 31 affirms the determination. In addition, as another example, in a case where a specific person is present in the acquired video, the CPU 31 affirms the determination. An example of the specific person is a patient serving as the subject H or a user who operates the imaging apparatus 10 or the like in a case of imaging an affected part, such as a radiologist. In addition, for example, in a case where the movement of any person and any object included in the acquired video is detected, the CPU 31 affirms the determination. In addition, as another example, for example, in a case where persons such as a patient serving as the subject H and a user included in the acquired video are recognized, and a movement of a predetermined specific person among the recognized persons is detected, the CPU 31 affirms the determination. Here, the subject H, the patient serving as the subject H, and the user are examples of "objects". In addition, the patient serving as the subject H and the user are examples of "persons".

In addition, as another example, in a case where a gesture, which indicates a turn-on instruction, of any person included in the video, a recognized predetermined specific person, or the like is detected, the CPU 31 affirms the determination. The gesture indicating the turn-on instruction is an example of a "specific movement". The association between the turn-on instruction of the light irradiation unit 14 and the gesture is set in advance by the user and is stored in, for example, the storage unit 33.

In the process of step S204, for example, the presence and movement of an object, a person, or the like included in the entire region of the range C imaged by the optical camera 13 may be a target of detection. Alternatively, the presence and movement of an object, a person, or the like included in a limited range such as a part of the range C, for example, a range corresponding to the radiation detector 15, the range R serving as the irradiation region of radiation, and the like, may be a target of detection. With the above configuration, it is possible to prevent the light from being unnecessarily turned on and turned off, compared to a case where the range in which the video is acquired is not limited.

In a case where the movement is detected in the process of step S204, the CPU 31 affirms the determination and executes the process of step S206. In a case where the movement is not detected in the process of step S204, the CPU 31 denies the determination and executes the process of step S202.

In step S206, the CPU 31 turns on the light irradiation unit 14.

In the process of step S208, the CPU 31 determines whether or not a turn-off instruction indicating an instruction to turn off the light irradiation unit 14 has been received by pressing a switch provided in the operation unit 35 or the like. In a case where the turn-off instruction is received, the CPU 31 affirms the determination and executes the process of step S218. In a case where the turn-off instruction is not received, the CPU 31 denies the determination and executes the process of step S210.

In the process of step S210, the CPU 31 determines whether or not the movement of the person or the object included in the acquired video is detected. The details of the movement of the person or the object included in the video are the same as the description of step S204, and thus the description thereof will be omitted.

In a case where the movement is detected in the process of step S210, the CPU 31 affirms the determination and executes the process of step S212. On the other hand, in a case where the movement is not detected, the CPU 31 denies the determination and executes the process of step S216.

In the process of step S212, the CPU 31 determines whether or not the turn-off instruction is received from the detected movement. For example, in a case where a gesture is detected as the movement in the process of step S210, in a case where the gesture indicates the turn-off instruction, the CPU 31 affirms the determination. Meanwhile, in a case where the movement of the person included in the video is detected, in a case where the movement is not a movement indicating the turn-off instruction, the determination is denied. The association between the turn-off instruction of the light irradiation unit 14 and the gesture is set in advance by the user and is stored in, for example, the storage unit 33.

In a case where the determination of the process of step S212 is affirmed, the CPU 31 executes the process of step S218. In a case where the determination of the process of step S212 is denied, the CPU 31 executes the process of step S214.

In the process of step S212, for example, the presence and movement of the object, the person, or the like included in the entire region of the range C imaged by the optical camera 13 may be a target of detection. Alternatively, the presence and movement of an object, a person, or the like included in a limited range such as a part of the range C, for example, a range corresponding to the radiation detector 15, the range R serving as the irradiation region of radiation, and the like, may be a target of detection. With the above configuration, it is possible to prevent the light from being unnecessarily turned on and turned off, compared to a case where the range in which the video is acquired is not limited.

In step S214, the CPU 31 resets the count of the timer of the light irradiation unit 14.

In step S216, the CPU 31 determines whether or not a predetermined time has elapsed since the start of turning on the light irradiation unit 14. An example of the predetermined time is a predetermined time, for example, 30 seconds. In a case where the predetermined time has elapsed, the CPU 31 affirms the determination and executes the process of step S218. In a case where the predetermined time has not elapsed, the CPU 31 denies the determination and executes the process of step S208.

In step S218, the CPU 31 turns off the light irradiation unit 14. While the process of Fig. 4 is being executed, the CPU 31 repeatedly executes the process shown in Fig. 5 while the control via the first mode is being continued.

As described above, according to the process of step S200 to step S218, the CPU 31 can control turning on and turning off the light irradiation unit 14 using the video acquired by the optical camera 13 in addition to the control turning on and turning off the light irradiation unit 14 via the user pressing the switch.

In the process of step S204, step S210, and step S212, in a case where the video includes a plurality of persons, the CPU 31 may be configured to detect a movement limited to a specific person such as the user and perform the determination. With the above configuration, in a case where the plurality of persons are included in the video, it is possible to prevent the light from being turned on or turned off by the movement other than the movement of the specific person.

Next, a detailed description of process of controlling the light irradiation unit 14 via the process of the second mode will be made with reference to Fig. 6. The process shown in Fig. 6 is executed by the CPU 31, for example, while the process of Fig. 4 is being executed. The process shown in Fig. 6 is not executed while the control via the first mode is continued in a case where the determination of step S104 is affirmed.

In step S300, the CPU 31 determines whether or not the turn-on instruction of the light irradiation unit 14 is received from the operation unit 35. In a case where an instruction to turn on the light irradiation unit 14 is given by pressing a button provided in the operation unit 35, or the like, the CPU 31 affirms the determination.

In a case where the CPU 31 affirms the determination, the CPU 31 executes the process of step S302. In a case where the CPU 31 denies the determination, the CPU 31 executes the process of step S300.

In step S302, the CPU 31 turns on the light irradiation unit 14.

In step S304, the CPU 31 determines whether or not the turn-off instruction of the light irradiation unit 14 is received. In a case where the turn-off instruction of the light irradiation unit 14 is received by pressing a button provided in the operation unit 35, or the like, the CPU 31 affirms the determination.

In a case where the CPU 31 affirms the determination, the CPU 31 executes the process of step S308. In a case where the CPU 31 denies the determination, the CPU 31 executes the process of step S306.

In step S306, the CPU 31 determines whether or not a predetermined time has elapsed since the start of turning on the light irradiation unit 14. The predetermined time is, for example, 30 seconds. In a case where the predetermined time has elapsed since the start of turning on the light irradiation unit 14, the CPU 31 affirms the determination of step S306.

In a case where the CPU 31 affirms the determination, the CPU 31 executes the process of step S308. In a case where the CPU 31 denies the determination, the CPU 31 executes the process of step S304.

In step S308, the CPU 31 controls the light irradiation unit 14 to be turned off. While the process of Fig. 4 is being executed, the process shown in Fig. 6 is repeatedly executed.

As described above, according to the process of steps S300 to S308, the CPU 31 can control turning on and turning off the light irradiation unit 14 by, for example, the operation of the operation unit 35 of the user. With the second mode, the CPU 31 does not control turning on and turning off the light irradiation unit 14 using the video acquired by the optical camera 13.

As described above, according to the process shown in Figs. 4 to 6, at least one of the turning on or turning off of the light irradiation unit 14 can be controlled using the video acquired by the optical camera 13 according to the user's settings. In addition, with the imaging apparatus 10 equipped with the optical camera 13, the user can control turning on and turning off the light irradiation unit 14 remotely without adding a dedicated input device such as an ultrasonic distance sensor, an optical distance sensor, and a voice input microphone.

In addition, according to the control of the light irradiation unit 14 based on the video (the control of the light irradiation unit 14 via the first mode), it is possible to automatically turn on the light according to the presence of a person, as compared with a case where the user turns on the light with the switch. In addition, according to the control of the light irradiation unit 14 based on the video (the control of the light irradiation unit 14 via the first mode), in a case where the light used in a case of determining the range in which the radiation is emitted is automatically turned off by the timer, the user can easily turn on the light as compared with a case where the user turns on the light again by using the switch.

In addition, according to the control of the light irradiation unit 14 based on the video (the control of the light irradiation unit 14 via the first mode), the user can easily control turning on and turning off the light remotely, as compared with a case where the user turns on the light with a switch and automatically turns off the light with a timer. In addition, according to the control of the light irradiation unit 14 based on the video (the control of the light irradiation unit 14 via the first mode), in a case where the plurality of persons are included in the video, it is possible to prevent the light from being turned on or turned off by the movement other than the movement of the specific person.

In addition, according to the control of the light irradiation unit 14 based on the video (the control of the light irradiation unit 14 via the first mode), it is possible to prevent the light from being unnecessarily turned on and turned off, compared to a case where the range in which the video is acquired is not limited.

In addition, with the configuration in which the first mode can be set as the control mode of the light irradiation unit 14 in the setting information, the user can select whether to control turning on and turning off the light using the acquired video or to control turning on and turning off the light without using the acquired video.

In addition to the above, a configuration may be adopted in which the timing of controlling turning on and turning off the light irradiation unit 14 in the first mode is appropriately changed by, for example, the user's settings.

For example, in a case where the imaging apparatus 10 is available for performing imaging, in other words, the imaging apparatus 10 is available for performing radiography, the CPU 31 may be configured to control turning on and turning off the light irradiation unit 14 in the first mode. The case in which the imaging apparatus 10 is available for performing imaging indicates, for example, a period from the registration of the setting information via the user in the console 30 to the start of the emission of the radiation. More specifically, the case indicates a period in which the process from step S102 to step S108 in Fig. 4 is executed. With the above-described configuration, it is possible to prevent the light from being unnecessarily turned on, compared to a case where the turning on and turning off of the light are controlled by the first mode at all times.

In addition, for example, in a case where a specific imaging menu is set in the imaging menu included in the setting information, the CPU 31 may be configured to control turning on and turning off the light irradiation unit 14 in the first mode. An example of the specific imaging menu is chest imaging, abdominal imaging, and the like. With the above-described configuration, it is possible to prevent the light from being unnecessarily turned on, compared to a case where the turning on and turning off of the light are controlled by the first mode at all times.

In addition, for example, in a case where the imaging method can be set in the imaging menu included in the setting information and the imaging method is a specific imaging method, the CPU 31 may be configured to control turning on and turning off the light irradiation unit 14 in the first mode. Examples of the imaging method include upright imaging without using a bed and decubitus imaging using a bed. For example, the user can set the light irradiation unit 14 to be controlled to be turned on and turned off in the first mode in a case of upright imaging. With the above-described configuration, it is possible to prevent the light from being unnecessarily turned on, compared to a case where the turning on and turning off of the light are controlled by the first mode at all times.

According to the present embodiment, it is possible to control turning on and turning off the light by using at least one of the video corresponding to the visible light image or the video of the depth image corresponding to the visible light image.

In any case, the light irradiation unit 14 is turned off during the emission of the radiation.

The process shown in Figs. 4, 5, and 6 has been described as being executed by the CPU 31 of the console 30, but the process with the information processing program 100 may be executed by the imaging apparatus 10. In a case where the imaging apparatus 10 executes the process, the imaging apparatus 10 comprises a controller capable of executing the information processing program 100.

In the present embodiment, the information processing program 100 has been described as being stored in the storage unit 33, but the information processing program 100 may be provided by a storage medium such as a CD-ROM, or may be downloaded via a network.

## Claims

1. An information processing apparatus (30) comprising:
a processor (31),
wherein the processor (31) is configured to:
acquire a real-time video with an imaging apparatus (13); and
control at least one of turning on or turning off light (14) used to determine an irradiation range of radiation according to a movement of an object (H) included in the acquired video.

2. The information processing apparatus (30) according to claim 1,
wherein, in a case where a presence of a person is detected as the movement of the object (H) from the acquired video, the processor (31) controls the light (14) to be turned on.

3. The information processing apparatus (30) according to claim 1,
wherein, in a case where a movement of a person is detected as the movement of the object (H) from the acquired video, the processor (31) controls the light (14) to be turned on.

4. The information processing apparatus (30) according to claim 1,
wherein, in a case where a specific movement of a person is detected as the movement of the object (H) from the acquired video, the processor (31) controls the light (14) to be at least one of turned on or turned off.

5. The information processing apparatus (30) according to claim 1,
wherein, in a case where a movement of a plurality of persons is detected as the movement of the object (H) from the acquired video, the processor (31) recognizes a specific movement of a specific person from among the plurality of persons, and
controls the light (14) to be at least one of turned on or turned off based on the recognized specific movement of the specific person.

6. The information processing apparatus (30) according to claim 1,
wherein the processor (31) is configured to:
limit a range in which the video is acquired to a size of an image receiving unit (15) for the radiation or to the irradiation range of the radiation; and
control turning on and turning off the light (14) based on the video acquired in the limited range.

7. The information processing apparatus (30) according to any one of claims 1 to 6,
wherein the processor (31) is configured to control turning on and turning off the light (14) based on a first mode in which the light (14) is turned on and turned off using the acquired video or on a second mode in which the light (14) is not turned on and turned off using the acquired video.

8. The information processing apparatus (30) according to claim 7,
wherein the processor (31) is configured to control turning on and turning off the light (14) via the first mode in a state in which radiography is possible.

9. The information processing apparatus (30) according to claim 7,
wherein the processor (31) is configured to control turning on and turning off the light (14) via the first mode in a case of radiography in a specific imaging menu.

10. The information processing apparatus (30) according to claim 7,
wherein the processor (31) is configured to control turning on and turning off the light (14) via the first mode in a case of radiography at an upright position.

11. The information processing apparatus (30) according to claim 7,
wherein the video acquired by the imaging apparatus (13) is a visible light image.

12. The information processing apparatus (30) according to claim 7,
wherein the video acquired by the imaging apparatus (13) is a visible light image and a depth image corresponding to the visible light image.

13. An information processing program (100) causing a computer (31) to execute a process comprising:
acquiring a real-time video with an imaging apparatus (13) ; and
controlling at least one of turning on or turning off light (14) used to determine an irradiation range of radiation according to a movement of an object (H) included in the acquired video.

14. An information processing method executed by a computer (31), the method comprising:
a step of acquiring a real-time video with an imaging apparatus (13); and
a step of controlling at least one of turning on or turning off light (14) used to determine an irradiation range of radiation according to a movement of an object included in the acquired video.

## Patentansprüche

1. Informationsverarbeitungsvorrichtung (30), umfassend:
einen Prozessor (31),
wobei der Prozessor (31) so konfiguriert ist, dass er:
ein Echtzeitvideo mit einer Bildgebungsvorrichtung (13) erfasst; und
mindestens eines von Einschalten und Ausschalten von Licht (14), das zum Bestimmen eines Bestrahlungsbereichs von Strahlung verwendet wird, gemäß einer Bewegung eines Objekts (H), das in dem erfassten Video enthalten ist, steuert.

2. Informationsverarbeitungsvorrichtung (30) nach Anspruch 1,
wobei in einem Fall, in dem ein Vorhandensein einer Person als die Bewegung des Objekts (H) aus dem erfassten Video detektiert wird, der Prozessor (31) das Licht (14) so steuert, dass es eingeschaltet wird.

3. Informationsverarbeitungsvorrichtung (30) nach Anspruch 1,
wobei in einem Fall, in dem eine Bewegung einer Person als die Bewegung des Objekts (H) aus dem erfassten Video detektiert wird, der Prozessor (31) das Licht (14) so steuert, dass es eingeschaltet wird.

4. Informationsverarbeitungsvorrichtung (30) nach Anspruch 1,
wobei in einem Fall, in dem eine spezifische Bewegung einer Person als die Bewegung des Objekts (H) aus dem erfassten Video detektiert wird, der Prozessor (31) das Licht (14) so steuert, dass es mindestens eines von eingeschaltet und ausgeschaltet wird.

5. Informationsverarbeitungsvorrichtung (30) nach Anspruch 1,
wobei in einem Fall, in dem eine Bewegung von mehreren Personen als die Bewegung des Objekts (H) aus dem erfassten Video detektiert wird, der Prozessor (31) eine spezifische Bewegung einer spezifischen Person unter den mehreren Personen erkennt, und
das Licht (14) auf der Grundlage der erkannten spezifischen Bewegung der spezifischen Person so steuert, dass es mindestens eines von eingeschaltet und ausgeschaltet wird.

6. Informationsverarbeitungsvorrichtung (30) nach Anspruch 1,
wobei der Prozessor (31) so konfiguriert ist, dass er:
einen Bereich, in dem das Video erfasst wird, auf eine Größe einer Bildempfangseinheit (15) für die Strahlung oder auf den Bestrahlungsbereich der Strahlung begrenzt; und
Einschalten und Ausschalten des Lichts (14) auf der Grundlage des in dem begrenzten Bereich erfassten Videos steuert.

7. Informationsverarbeitungsvorrichtung (30) nach einem der Ansprüche 1 bis 6, wobei der Prozessor (31) so konfiguriert ist, dass er Einschalten und Ausschalten des Lichts (14) auf der Grundlage eines ersten Modus, bei dem das Licht (14) unter Verwendung des erfassten Videos ein- und ausgeschaltet wird, oder eines zweiten Modus, bei dem das Licht (14) nicht unter Verwendung des erfassten Videos ein- und ausgeschaltet wird, steuert.

8. Informationsverarbeitungsvorrichtung (30) nach Anspruch 7,
wobei der Prozessor (31) so konfiguriert ist, dass er Einschalten und Ausschalten des Lichts (14) via den ersten Modus in einem Zustand, in dem Röntgenaufnahme möglich ist, steuert.

9. Informationsverarbeitungsvorrichtung (30) nach Anspruch 7,
wobei der Prozessor (31) so konfiguriert ist, dass er Einschalten und Ausschalten des Lichts (14) via den ersten Modus in einem Fall von Röntgenaufnahme in einem spezifischen Bildgebungsmenü steuert.

10. Informationsverarbeitungsvorrichtung (30) nach Anspruch 7,
wobei der Prozessor (31) so konfiguriert ist, dass er Einschalten und Ausschalten des Lichts (14) via den ersten Modus in einem Fall von Röntgenaufnahme an einer aufrechten Position steuert.

11. Informationsverarbeitungsvorrichtung (30) nach Anspruch 7,
wobei das von der Bildgebungsvorrichtung (13) erfasste Video ein Bild von sichtbarem Licht ist.

12. Informationsverarbeitungsvorrichtung (30) nach Anspruch 7,
wobei das von der Bildgebungsvorrichtung (13) erfasste Video ein Bild von sichtbarem Licht und ein Tiefenbild, das dem Bild von sichtbarem Licht entspricht, ist.

13. Informationsverarbeitungsprogramm (100), das einen Computer (31) veranlasst, einen Prozess auszuführen, der umfasst:
Erfassen eines Echtzeitvideos mit einer Bildgebungsvorrichtung (13); und
Steuern von mindestens einem von Einschalten und Ausschalten von Licht (14), das zum Bestimmen eines Bestrahlungsbereichs von Strahlung verwendet wird, gemäß einer Bewegung eines Objekts (H), das in dem erfassten Video enthalten ist.

14. Informationsverarbeitungsverfahren, das von einem Computer (31) ausgeführt wird, wobei das Verfahren umfasst:
einen Schritt des Erfassens eines Echtzeitvideos mit einer Bildgebungsvorrichtung (13); und
einen Schritt des Steuerns von mindestens einem von Einschalten und Ausschalten von Licht (14), das zum Bestimmen eines Bestrahlungsbereichs von Strahlung verwendet wird, gemäß einer Bewegung eines Objekts, das in dem erfassten Video enthalten ist.

## Revendications

1. Appareil de traitement d'informations (30) comprenant :
un processeur (31),
dans lequel le processeur (31) est configuré pour :
acquérir une vidéo en temps réel à l'aide d'un appareil d'imagerie (13) ; et
contrôler au moins l'un de l'activation ou de la désactivation d'une lumière (14) utilisée pour déterminer une plage d'irradiation de rayonnement en fonction d'un mouvement d'un objet (H) inclus dans la vidéo acquise.

2. Appareil de traitement d'informations (30) selon la revendication 1,
dans lequel, dans un cas où une présence d'une personne est détectée comme le mouvement de l'objet (H) à partir de la vidéo acquise, le processeur (31) contrôle l'activation de la lumière (14).

3. Appareil de traitement d'informations (30) selon la revendication 1,
dans lequel, dans un cas où un mouvement d'une personne est détecté comme le mouvement de l'objet (H) à partir de la vidéo acquise, le processeur (31) contrôle l'activation de la lumière (14).

4. Appareil de traitement d'informations (30) selon la revendication 1,
dans lequel, dans un cas où un mouvement spécifique d'une personne est détecté comme le mouvement de l'objet (H) à partir de la vidéo acquise, le processeur (31) contrôle au moins l'une de l'activation ou de la désactivation de la lumière (14).

5. Appareil de traitement d'informations (30) selon la revendication 1,
dans lequel, dans un cas où un mouvement d'une pluralité de personnes est détecté comme le mouvement de l'objet (H) à partir de la vidéo acquise, le processeur (31) reconnaît un mouvement spécifique d'une personne spécifique parmi la pluralité de personnes, et
contrôle la lumière (14) de manière à ce qu'elle soit au moins activée ou désactivée sur la base du mouvement spécifique reconnu de la personne spécifique.

6. Appareil de traitement d'informations (30) selon la revendication 1,
dans lequel le processeur (31) est configuré pour :
limiter une plage dans laquelle la vidéo est acquise à une taille d'une unité de réception d'images (15) pour le rayonnement ou à la plage d'irradiation du rayonnement ; et
contrôler l'activation et la désactivation de la lumière (14) sur la base de la vidéo acquise dans la plage limitée.

7. Appareil de traitement d'informations (30) selon l'une quelconque des revendications 1 à 6,
dans lequel le processeur (31) est configuré pour contrôler l'activation et la désactivation de la lumière (14) sur la base d'un premier mode dans lequel la lumière (14) est activée et désactivée en utilisant la vidéo acquise ou d'un deuxième mode dans lequel la lumière (14) n'est pas activée et désactivée en utilisant la vidéo acquise.

8. Appareil de traitement d'informations (30) selon la revendication 7,
dans lequel le processeur (31) est configuré pour contrôler l'activation et la désactivation de la lumière (14) via le premier mode dans un état où la radiographie est possible.

9. Appareil de traitement d'informations (30) selon la revendication 7,
dans lequel le processeur (31) est configuré pour contrôler l'activation et la désactivation de la lumière (14) via le premier mode dans un cas de radiographie dans un menu d'imagerie spécifique.

10. Appareil de traitement d'informations (30) selon la revendication 7,
dans lequel le processeur (31) est configuré pour contrôler l'activation et la désactivation de la lumière (14) via le premier mode dans un cas de radiographie dans une position debout.

11. Appareil de traitement d'informations (30) selon la revendication 7,
dans lequel la vidéo acquise par l'appareil d'imagerie (13) est une image en lumière visible.

12. Appareil de traitement d'informations (30) selon la revendication 7,
dans lequel la vidéo acquise par l'appareil d'imagerie (13) est une image en lumière visible et une image de profondeur correspondant à l'image en lumière visible.

13. Programme de traitement d'informations (100) amenant un ordinateur (31) à exécuter un processus comprenant :
acquérir une vidéo en temps réel à l'aide d'un appareil d'imagerie (13) ; et
contrôler au moins l'un de l'activation ou de la désactivation d'une lumière (14) utilisée pour déterminer une plage d'irradiation de rayonnement en fonction d'un mouvement d'un objet (H) inclus dans la vidéo acquise.

14. Procédé de traitement d'informations exécuté par un ordinateur (31), le procédé comprenant :
une étape consistant à acquérir une vidéo en temps réel à l'aide d'un appareil d'imagerie (13) ; et
une étape consistant à contrôler au moins l'un de l'activation ou de la désactivation d'une lumière (14) utilisée pour déterminer une plage d'irradiation de rayonnement en fonction d'un mouvement d'un objet inclus dans la vidéo acquise.
